(19) Europäisches Patentamt / European Patent Office / Office européen des brevets

(11) **EP 3 718 415 A1**

(12) **EUROPEAN PATENT APPLICATION**
published in accordance with Art. 153(4) EPC

(43) Date of publication:
07.10.2020 Bulletin 2020/41

(51) Int Cl.:
*A23L 5/20* (2016.01)　　　*A23C 9/00* (2006.01)
*A23C 9/13* (2006.01)

(21) Application number: 18884296.7

(22) Date of filing: 11.10.2018

(86) International application number:
PCT/CN2018/109769

(87) International publication number:
WO 2019/105130 (06.06.2019 Gazette 2019/23)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.11.2017 CN 201711235972

(71) Applicant: Inner Mongolia Yili Industrial Group
Co., Ltd.
Hohhot, Inner Mongolia 010110 (CN)

(72) Inventors:
• CHANG, Pengfei
Hohhot
Inner Mongolia 010110 (CN)
• LIU, Biao
Hohhot
Inner Mongolia 010110 (CN)
• KONG, Xiaoyu
Hohhot
Inner Mongolia 010110 (CN)

(74) Representative: Colombo, Stefano Paolo et al
Marchi & Partners S.r.l.
Via Vittor Pisani, 13
20124 Milano (IT)

(54) **HYDROLYSED PROTEIN DEBITTERING COMPOSITION AND PRODUCT, PREPARATION, AND APPLICATION THEREOF**

(57) The present invention provides a composition for debittering hydrolyzed proteins and a product, preparation and use thereof. The composition comprises a short chain fatty acid, a medium chain fatty acid and a long chain fatty acid, wherein, based on total mass of the short chain fatty acid, the medium chain fatty acid and the long chain fatty acid which is 100%, the medium chain fatty acid is present in an amount of 0.2-4.7%, the long chain fatty acid is present in an amount of 95.1-99.4%, and the remaining is the short chain fatty acid. The present invention can effectively reduce the bitter taste of hydrolyzed proteins and is widely used.

EP 3 718 415 A1

## Description

## Technical field

The present invention relates to the field of foods, and in particular to a composition for debittering hydrolyzed proteins and a product, preparation and use thereof.

## Background art

Hydrolysis of a protein with an enzyme is a common way to improve the application properties of the protein in the field of foods. The obtained hydrolyzed protein is a high-quality protein resource, and has been applied in general foods and special nutritional foods, such as foods for the elderly and patients with poor gastrointestinal absorption, low-antigenic infant formula foods, sports foods, and diet foods. However, during enzymatic hydrolysis of a protein, bitter taste at different levels will be produced due to the exposure of hydrophobic groups thereof, limiting the development and application of products of hydrolyzed protein. Accordingly, how to reduce the bitter taste of hydrolyzed proteins has become the main technical bottleneck for development thereof as foods.

At present, there are many studies on the removal of bitter taste of hydrolyzed proteins at home and abroad, and there are many methods for removing the bitter taste, and the application fields and debittering effects thereof are different from each other. Physicochemical removal of bitter taste has been widely applied, including separation, extraction, adsorption, masking, protein modification and acid-base hydrolysis etc.

Removal of bitter taste by selective separation: the principle of selective separation is to selectively remove the highly hydrophobic polypeptides in the protein hydrolysate by using activated carbon, resins or solvents, according to the difference in hydrophobicity among different polypeptide components in the protein hydrolysate. However, after activated carbon adsorbs hydrophobic amino acids, nearly 26% of protein nitrogen will be lost, many of which are essential amino acids, which affects the amino acid composition of the peptides and reduces the nutritional value of the protein hydrolysate.

Extraction with azeotropic isobutanol: a mixture of 72.8% isobutanol and 27.2% water is referred to as azeotrope isobutanol. A good effect can be achieved by extracting an enzymatic protein hydrolyzate with azeotrope isobutanol, which is a typical method commonly used for removing bitter complexes. However, in order to achieve the purpose of completely removing bitter taste, 5%-10% of protein hydrolysate will be lost, greatly affecting the nutritional value thereof.

Treatment with a hydrophobic chromatographic column: the pH value of an aqueous solution of a protein hydrolyzate is adjusted to 7, loaded on a hexyl-sepharose gel column, and eluted at room temperature. Then the bitter peptides containing hydrophobic amino acids would bound to the gel support to fulfill debittering purpose. However, the method cannot be applied to commercial production due to the complexity of the process and high technical requirements.

Masking: masking means adding certain substances that can mask the bitter taste of protein hydrolysates to a bitter protein hydrolysate solution to reduce the bitter taste. For example, during the hydrolysis of a protein, addition of polyphosphoric acid can successfully mask the bitter taste caused by hydrolysis of casein, and addition of gelatin can also achieve similar effect, but the effect is not as good as that resulted from addition of glycine. Cross-linked starch can conceal bitter taste inside the molecular structure of starch, thereby preventing them from contacting with taste buds to mask the bitter taste. To achieve this effect, a mixture of the starch and a bitter peptide must be heated. Combining a bitter peptide with concentrated whey protein, skim milk and soybean also has effects of removing or masking bitter taste due to the affinity between proteins, amino acids and peptides. It is known in the art that bitter taste is also reduced after mixing with an acid, but there is a disadvantage that sour taste is produced. An acidic solution of taurine can reduce bitter taste of amino acids without producing sour taste, but the bitter taste can only be masked when the acidic solution is added in a large amount.

The above methods have obvious limitations when applied to debitter hydrolyzed proteins, and have not significantly broken the limitation of the bitter taste of hydrolyzed proteins, and the debittering effects thereof are not significant. Therefore, it is extremely necessary to invent a debittering composition which is effective to reduce the bitter taste of hydrolyzed proteins and can be widely applied.

## Contents of the invention

It is an object of the present invention to provide a composition for debittering hydrolyzed proteins.

It is another object of the present invention to provide a debittered hydrolyzed protein-containing composition.

It is further object of the present invention to provide a debittered hydrolyzed protein solution.

It is further object of the present invention to provide a method for producing the debittered hydrolyzed protein solution.

It is further object of the present invention to provide a debittered hydrolyzed protein powder.

It is still another object of the present invention to provide a use of the composition for debittering hydrolyzed proteins for debittering a hydrolyzed protein.

In order to achieve the above objects, in one aspect, the present invention provides a composition for debittering hydrolyzed proteins comprising a short chain fatty acid, a medium chain fatty acid and a long chain fatty acid, wherein, based on the total mass of the short chain fatty acid, the medium chain fatty acid and the long chain fatty acid which is 100%, the medium chain fatty acid is present in an amount of 0.2-4.7%, the long chain fatty acid is present in an amount of 95.1-99.4%, and the remaining is the short chain fatty acid.

According to some embodiments of the present invention, the short-chain fatty acid is a linear or branched fatty acid having 1 to 5 carbon atoms; the medium-chain fatty acid is a linear or branched fatty acid having 6 to 12 carbon atoms; and the long chain fatty acid is a linear or branched fatty acid having a carbon number of greater than 12.

According to some embodiments of the present invention, the composition for debittering hydrolyzed proteins is prepared by mixing one or more of vegetable oil, animal fat, and 1,3-dioleoyl-2-palmitoyl triglyceride.

According to some embodiments of the present invention, the vegetable oil is one selected from the group consisting of soybean oil, corn oil, sunflower seed oil, walnut oil, canola oil, palm oil, medium chain triglyceride, soybean phospholipid, rapeseed oil, and a mixture thereof.

According to some embodiments of the present invention, the animal fat is one selected from the group consisting of anhydrous butter, docosahexaenoic acid, arachidonic acid, and a mixture thereof.

A person skilled in the art can determine the amount of each of the above-mentioned substances required to formulate the composition for debittering hydrolyzed proteins on the basis of the amount of fatty acids in each of the substances.

In another aspect, the present invention further provides a debittered hydrolyzed protein-containing composition, wherein the debittered hydrolyzed protein-containing composition comprises the following components in the following parts by weight: 2-6 parts of the debittering composition of the present invention, 2-36 parts of hydrolyzed protein, 0-1 part of phospholipid, and 0-0.4 parts of an acidity regulator.

According to some embodiments of the present invention, the hydrolyzed protein is present in a quantity of 6-30 parts.

According to some embodiments of the present invention, the hydrolyzed protein has a degree of hydrolysis of from 5 to 45.

According to some embodiments of the present invention, the hydrolyzed protein has a degree of hydrolysis of from 6 to 20.

According to some embodiments of the present invention, the acidity regulator is one selected from the group consisting of citric acid, sodium citrate, tartaric acid, potassium citrate, malic acid, and a mixture thereof.

In another aspect, the present invention provides a debittered hydrolyzed protein solution, wherein the debittered hydrolyzed protein solution comprises the following components in the following parts by weight: 4-43.4 parts of the debittered hydrolyzed protein-containing composition according to any one of the embodiment of the present invention, and water as the balance.

In another aspect, the present invention provides a method for producing the debittered hydrolyzed protein solution, wherein the method comprises the following steps of: the components are mixed uniformly, subjected to a high speed shearing, then homogenized, and cooled to obtain the debittered hydrolyzed protein solution.

According to some embodiments of the present invention, the high speed shearing is carried out at 10000-20000 r/min.

According to some embodiments of the present invention, the high speed shearing is carried out at a temperature of 40-50°C.

According to some embodiments of the present invention, the high speed shearing is carried out for 5-20 minutes.

According to some embodiments of the present invention, the homogenization is carried out at a pressure of 30-100 MPa.

According to some embodiments of the present invention, the method further comprises a step of drying treatment to obtain a debittered hydrolyzed protein powder, after the debittered hydrolyzed solution is obtained by cooling.

According to some embodiments of the present invention, the drying treatment is spray drying, fluidized bed drying, rotary flash drying, microwave drying, or freeze drying.

In a further aspect, the present invention also provides a debittered hydrolyzed protein powder obtained by the above preparation method.

In still another aspect, the present invention also provides a use of the composition for debittering hydrolyzed proteins for debittering a hydrolyzed protein.

In still another aspect, the present invention also provides a use of the composition for debittering hydrolyzed proteins in the manufacture of a milk powder or a formula food for a special medical use.

According to some embodiments of the present invention, the milk powder is an infant milk powder or a milk powder for middle-aged and old peoples.

According to some embodiments of the present invention, the formula food for a special medical use is an infant formula food for a special medical use.

The infant formula food for a special medical use refers to a powdered or liquid formula food designed to meet the nutritional needs of infants with special medical conditions such as special disorders, diseases or medical conditions. Under the guidance of a physician or clinical dietitian, when the infant formula food is consumed alone or in combination with other foods, the energy and nutrients thereof can meet the growth and development needs of infants from 0 months to 6 months with special medical conditions.

In still another aspect, the present invention further provides an infant formula milk powder comprising a hydrolyzed protein, wherein, in the infant formula milk powder, the protein content of the infant formula is 0.45-1.2 g/100 kJ, the fat content is 0.7-1.4 g/100 kJ, and the carbohydrate content is 2.2-3.3 g/100 kJ.

According to some embodiments of the present invention, the infant formula milk powder at least comprises the following components in following percentages: 0.1%-40% of a hydrolyzed protein component, and 10%-28% of the composition for debittering hydrolyzed proteins according to any one of the embodiment of the present invention, wherein the proteins are provided by the hydrolyzed protein and the milk component of the infant formula milk powder.

It can be understood that the infant formula milk powder further comprises additional components as specified in the corresponding standards, such as milk component.

Each of the additional components can be added in an amount in accordance with, such as the corresponding standards for infant formula milk powder in the prior art.

The milk component may be one selected from the group consisting of whole milk powder, skimmed milk powder, raw milk, and a mixture thereof.

According to some embodiments of the present invention, the hydrolyzed protein component is one selected from the group consisting of hydrolyzed whey protein, hydrolyzed milk protein, hydrolyzed casein, and a mixture thereof.

According to some embodiments of the present invention, the hydrolyzed protein is present in an amount of 2%-15%.

According to some embodiments of the present invention, the infant formula milk powder further comprises a non-hydrolyzed protein, and the proteins are provided by the hydrolyzed protein, the non-hydrolyzed protein and the milk component of the infant formula milk powder.

According to some embodiments of the present invention, the non-hydrolyzed protein is a whey protein.

In still another aspect, the present invention also provides a milk powder comprising a hydrolyzed protein for middle-aged and old peoples, wherein, the milk powder for middle-aged and old peoples comprises 16%-65% of proteins or 34%-69% of milk-solids-not-fat.

According to some embodiments of the present invention, the milk powder for middle-aged and old peoples at least comprises the following components in following percentages: 0.1%-40% of a hydrolyzed protein component, and 5-28% of the composition for debittering hydrolyzed proteins according to any one of the embodiment of the present invention, wherein the proteins are provided by the hydrolyzed protein and the milk component of the milk powder for middle-aged and old peoples.

It can be understood that the milk powder for middle-aged and old peoples further comprises additional components as specified in the corresponding standards, such as milk component.

Each of the additional components can be added in an amount in accordance with, such as the corresponding standards for milk powder for middle-aged and old peoples in the prior art.

The milk component may be one selected from the group consisting of whole milk powder, skimmed milk powder, raw milk and a mixture thereof.

According to some embodiments of the present invention, the hydrolyzed protein component is one selected from the group consisting of hydrolyzed whey protein, hydrolyzed milk protein, hydrolyzed casein, and a mixture thereof.

According to some embodiments of the present invention, the hydrolyzed protein is present in an amount of 2%-15%.

According to some embodiments of the present invention, the milk powder for middle-aged and old peoples further comprises a non-hydrolyzed protein, and the proteins are provided by the hydrolyzed protein, the non-hydrolyzed protein and milk component of the infant formula milk powder.

According to some embodiments of the present invention, the non-hydrolyzed protein is a whey protein.

In still another aspect, the present invention also provides an infant formula food for a special medical use, wherein, in the infant formula food for a special medical use, the protein content of the infant formula is 0.45-1.0 g/100 kJ, the fat content is 1.0-1.4 g/100 kJ, and the carbohydrate content is 2.2-3.3 g/100 kJ.

According to some embodiments of the present invention, the infant formula food for a special medical use at least comprises the following components in following percentages: 13-55% of a hydrolyzed protein component, and 15-28% of the composition for debittering hydrolyzed proteins according to any one of the embodiment of the present invention.

According to some embodiments of the present invention, the hydrolyzed protein component is one selected from the group consisting of hydrolyzed whey protein, hydrolyzed milk protein, hydrolyzed casein, and a mixture thereof.

To sum up, the present invention provides a composition for debittering hydrolyzed proteins and a product, preparation and use thereof. The debittering composition according to the present invention has the following advantages.

The present invention relates to a composition for debittering hydrolyzed proteins: the fats and oils are reasonably proportioned, and the amounts of medium-chain and long-chain fatty acids (based on the weight of total fatty acids) are adjusted, wherein the medium-chain fatty acid is present in an amount of 0.2-4.7% and the long-chain fatty acid is present in an amount of 95.1-99.4%. The present invention can effectively reduce the bitter taste of hydrolyzed proteins and is widely used.

## Specific mode for carrying out the invention

The implements and beneficial effects of the present invention are described in detail below by specific examples, which are intended to help a reader to understand the essence and characteristics of the present invention well but not to limit the scope of the present invention.

Evaluation criteria: the evaluation team consisted of 30 persons who are sensitive to bitter taste (for the milk powder containing a hydrolyzed protein for middle-aged and old peoples, the age of the evaluation team was limited to 45-55 years when the sensory evaluation was performed). First of all, the samples of the product in each of the examples were heated in a 40°C water bath for 30 minutes, and then the bitter taste thereof was scored by a 10-point scaling method, wherein 1-3 scores indicate almost no bitter taste, 3-5 scores indicate slight bitter taste, 5-7 scores indicate moderate bitter taste, 7-9 scores indicate obvious bitter taste, and 9-10 scores indicate strong bitter tast. A blank control was employed, and 3 parallel tests were carried out for each treatment. The score of bitter taste before treatment was set to x, the score of bitter taste after treatment was set to y, and

the debittering rate $= \dfrac{x-y}{x} \times 100\%$ .

## Example 1

Based on the total weight of oils, 28 parts of soybean oil, 10 parts of corn oil, 54 parts of sunflower seed oil, 1 part of anhydrous butter, 1 part of phospholipid and 6 parts of docosahexaenoic acid were mixed to achieve a desired requirement, that is, the medium-chain fatty acid was present in an amount of 0.557%, the long-chain fatty acid was present in an amount of 99.389%, and the short-chain fatty acid was present of an amount of 0.054%, with respect to the amount of total fatty acids, whereby a debittering composition was obtained.

4 parts of the above composition for debittering hydrolyzed proteins, 17 parts of a hydrolyzed whey protein having a degree of hydrolysis of 15.2, 0.1 parts of dipotassium hydrogen phosphate and water as the balance were weighed. The above materials were mixed, stirred uniformly, and sheared in a shearing machine at high speed, wherein the shearing temperature was of 45°C, and the shearing time was of 20 minutes. The sheared feed liquid was homogenized in a homogenizer at a homogenizing pressure of 80 MPa and cooled to obtain a debittered hydrolyzed protein solution. The resulting hydrolyzed protein was scored for sensory evaluation, and the experimental results showed that the debittering rate of the hydrolyzed protein was 68.31%.

## Example 2

Based on the total weight of oils, 23 parts of sunflower seed oil, 47 parts of 1,3-dioleoyl-2-palmitoyl triglyceride, 8 parts of corn oil, 15 parts of soybean oil, 1 part of anhydrous butter, 1 part of phospholipid and 5 parts of docosahexaenoic acid were mixed to achieve a desired requirement, that is, the medium-chain fatty acid was present in an amount of 1.022%, the long-chain fatty acid was present in an amount of 98.925%, and the short-chain fatty acid was present of an amount of 0.053%, with respect to the amount of total fatty acids, whereby a debittering composition was obtained.

5 parts of the above composition for debittering hydrolyzed proteins, 17 parts of a hydrolyzed whey protein having a degree of hydrolysis of 22.5, 0.1 parts of sodium citrate and water as the balance were weighed. The above materials were mixed, stirred uniformly, and sheared in a shearing machine at high speed, wherein the shearing temperature was of 40°C, and the shearing time was of 15 minutes. The sheared feed liquid was homogenized in a homogenizer at a homogenizing pressure of 100 MPa and cooled to obtain a debittered hydrolyzed whey protein solution. The resulting hydrolyzed protein was scored for sensory evaluation, and the experimental results showed that the debittering rate of the hydrolyzed protein was 71.95%.

## Example 3

Based on the total weight of oils, 25 parts of walnut oil, 46 parts of 1,3-dioleoyl-2-palmitoyl triglyceride, 8 parts of sunflower seed oil, 13 parts of corn oil, 4 parts of medium chain triglyceride, 1 part of phospholipid and 3 parts of docosahexaenoic acid were mixed to achieve a desired requirement, that is, the medium-chain fatty acid was present in an amount of 4.7%, the long-chain fatty acid was present in an amount of 95.103%, and the short-chain fatty acid was present of an amount of 0.197%, with respect to the amount of total fatty acids, whereby a debittering composition was obtained.

2 parts of the above composition for debittering hydrolyzed proteins, 6 parts of a hydrolyzed whey protein having a degree of hydrolysis of 15.5, 0.1 parts of potassium citrate and water as the balance were weighed. The above materials were mixed, stirred uniformly, and sheared in a shearing machine at high speed, wherein the shearing temperature was of 40°C, and the shearing time was of 15 minutes. The sheared feed liquid was homogenized in a homogenizer at a homogenizing pressure of 30 MPa and cooled to obtain a debittered hydrolyzed whey protein solution. The resulting hydrolyzed protein was scored for sensory evaluation, and the experimental results showed that the debittering rate of the hydrolyze protein was 70.27%.

## Example 4

Based on the total weight of oils, 45 parts of canola oil, 33 parts of palm oil, 11 parts of soybean oil, 3 parts of anhydrous butter, 2 parts of phospholipid and 6 parts of docosahexaenoic acid were mixed to achieve a desired requirement, that is, the medium-chain fatty acid was present in an amount of 0.2%, the long-chain fatty acid was present in an amount of 98.943%, and the short-chain fatty acid was present of an amount of 0.857%, with respect to the amount of total fatty acids, whereby a debittering composition was obtained.

6 parts of the above composition for debittering hydrolyzed proteins, 36 parts of a hydrolyzed whey protein having a degree of hydrolysis of 15.5, 0.2 parts of sodium citrate, 0.2 parts of potassium citrate and water as the balance were weighed. The above materials were mixed, stirred uniformly, and sheared in a shearing machine at high speed, wherein the shearing temperature was of 40°C, and the shearing time was of 15 minutes. The sheared feed liquid was homogenized in a homogenizer at a homogenizing pressure of 50 MPa and cooled to obtain a debittered hydrolyzed whey protein solution. The resulting hydrolyzed protein was scored for sensory evaluation, and the experimental results showed that the debittering rate of the hydrolyzed protein was 78.67%.

**Example 5**

Based on the total weight of oils, 60 parts of canola oil, 80 parts of corn oil, 50 parts of sunflower seed oil, 1 part of anhydrous butter, and 2 parts of phospholipid were mixed to achieve a desired requirement, that is, the medium-chain fatty acid was present in an amount of 0.27%, the long-chain fatty acid was present in an amount of 99.303%, and the short-chain fatty acid was present of an amount of 0.427%, with respect to the amount of total fatty acids, whereby a debittering composition was obtained. According to a formula of infant formula milk powder, to the debittering composition, 30 parts of a hydrolyzed whey protein powder having a degree of hydrolysis of 12.0, 55 parts of a whey protein powder, 150 parts of skimmed milk powder, 440 parts of lactose, 40 parts of oligo-fructose, 20 parts of oligo-galactose, and 10 parts of compound nutrients, were added. The above materials were mixed and formulated into a liquid milk having a concentration of 20%. The liquid milk was homogenized at a homogenizing pressure of 20 MPa and concentrated, and the concentrated liquid milk having a concentration of 39.5%. The concentrated liquid milk was subjected to spray drying, wherein the air inlet temperature was 110°C and the outlet air temperature was 80°C during drying, and the powder was discharged to obtain a debittered infant formula milk powder. The resulting hydrolyzed protein was scored for sensory evaluation, and the experimental results showed that the debittering rate of the hydrolyzed protein was 71.38%.

**Example 6**

Based on the total weight of oils, 30 parts of soybean oil, 14 parts of corn oil, 60 parts of sunflower seed oil, 10 parts of anhydrous butter, and 2 parts of phospholipids were mixed to achieve a desired requirement, that is, the medium-chain fatty acid was present in an amount of 0.327%, the long-chain fatty acid was present in an amount of 99.454%, and the short-chain fatty acid was present of an amount of 0.219%, with respect to the amount of total fatty acids, whereby a debittering composition was obtained. According to an infant formula milk powder for middle-aged and old peoples, to the debittering composition, 40 parts of a hydrolyzed whey protein powder having a degree of hydrolysis of 15.3, 160 parts of skimmed milk powder, 480 parts of lactose, 40 parts of oligo-fructose, 20 parts of oligo-galactose, and 5 parts of compound nutrients, were added. The above materials were mixed and formulated into a liquid milk having a concentration of 20%. The liquid milk was homogenized at a homogenizing pressure of 25 MPa and concentrated, and the concentrated liquid milk having a concentration of 40.5%. The concentrated liquid milk was subjected to spray drying, wherein the air inlet temperature was 110°C and the outlet air temperature was 80°C during drying, and the powder was discharged to obtain a debittered formula milk powder for middle-aged and old peoples. The resulting hydrolyzed protein was scored for sensory evaluation, and the experimental results showed that the debittering rate of the hydrolyzed protein was 72.34%.

**Example 7**

Based on the total weight of oils, 90 parts of 1,3-dioleoyl-2-palmitoyl triglyceride, 70 parts of corn oil, 100 parts of sunflower seed oil, and 2 parts of phospholipids were mixed to achieve a desired requirement, that is, the medium-chain fatty acid was present in an amount of 0.284%, the long-chain fatty acid was present in an amount of 99.531%, and the short-chain fatty acid was present of an amount of 0.185%, with respect to the amount of total fatty acids, whereby a debittering composition was obtained. According to a formula of infant formula milk powder for a special medical use, to the debittering composition, 140 parts of a hydrolyzed whey protein powder having a degree of hydrolysis of 15.3, 500 parts of lactose and 10 parts of compound nutrients, were added. The above materials were mixed and formulated into a liquid milk having a concentration of 20%. The liquid milk was homogenized at a homogenizing pressure of 25 MPa and concentrated, and the concentrated liquid milk having a concentration of 40%. The concentrated liquid milk was subjected to spray drying, wherein the air inlet temperature was 110°C and the outlet air temperature was 80°C during drying, and the powder was discharged to obtain an infant formula milk powder containing a debittered partially hydrolyzed whey protein. The resulting hydrolyzed protein was scored for sensory evaluation, and the experimental results showed that the debittering rate of the hydrolyzed protein was 80.11%.

**Comparative Example 1**

Based on the total weight of oils, 42 parts of sunflower seed oil, 49 parts of rapeseed oil, 8 parts of anhydrous butter, and 1 part of phospholipid were mixed to achieve that, the medium-chain fatty acid was present in an amount of 0.143%, the long-chain fatty acid was present in an amount of 99.621%, and the short-chain fatty acid was present of an amount of 0.236%, with respect to the amount of total fatty acids, whereby a debittering composition was obtained.

4 parts of the above composition for debittering hydrolyzed proteins, 17 parts of a hydrolyzed whey protein having a degree of hydrolysis of 15.2, 0.1 parts of dipotassium hydrogen phosphate and water as the balance were weighed. The above materials were mixed, stirred uniformly, and sheared in a shearing machine at high speed, wherein the shearing temperature was of 45°C, and the shearing time was of 20 minutes. The sheared feed liquid was homogenized in a homogenizer at a homogenizing pressure of 80 MPa and cooled to obtain a debittered hydrolyzed protein solution. The resulting hydrolyzed protein was scored for sensory evaluation, and the experimental results showed that the debittering rate of the hydrolyzed protein was 28.69%.

**Comparative Example 2**

Based on the total weight of oils, 28 parts of sunflower seed oil, 25 parts of medium chain triglyceride, 8 parts of corn oil, 25 parts of soybean oil, 2 parts of anhydrous butter, 2 parts of phospholipid and 10 parts of docosahexaenoic acid were mixed to achieve that, the medium-chain fatty acid was present in an amount of 5.148%, the long-chain fatty acid was present in an amount of 93.997%, and the short-chain fatty acid was present of an amount of 0.855%, with respect to the amount of total fatty acids, whereby a debittering composition was obtained.

5 parts of the above composition for debittering hydrolyzed proteins, 17 parts of a hydrolyzed whey protein having a degree of hydrolysis of 22.5, 0.1 parts of sodium citrate and water as the balance were weighed. The above materials were mixed, stirred uniformly, and sheared in a shearing machine at high speed, wherein the shearing temperature was of 40°C, and the shearing time was of 15 minutes. The sheared feed liquid was homogenized in a homogenizer at a homogenizing pressure of 100 MPa and cooled to obtain a debittered hydrolyzed protein solution. The resulting hydrolyzed protein was scored for sensory evaluation, and the experimental results showed that the debittering rate of the hydrolyzed protein was 24.01%.

**Claims**

1. A composition for debittering hydrolyzed proteins comprising a short chain fatty acid, a medium chain fatty acid and a long chain fatty acid, wherein, based on total mass of the short chain fatty acid, the medium chain fatty acid and the long chain fatty acid which is 100%, the medium chain fatty acid is present in an amount of 0.2-4.7%, the long chain fatty acid is present in an amount of 95.1-99.4%, and the remaining is the short chain fatty acid.

2. The debittering composition according to claim 1, wherein, the short-chain fatty acid is a linear or branched fatty acid having 1 to 5 carbon atoms; the medium-chain fatty acid is a linear or branched fatty acid having 6 to 12 carbon atoms; and the long chain fatty acid is a linear or branched fatty acid having a carbon number of greater than 12.

3. The debittering composition according to claim 1, wherein, the composition for debittering hydrolyzed proteins is prepared by mixing one or more of vegetable oil (preferably, one selected from the group consisting of soybean oil, corn oil, sunflower seed oil, walnut oil, canola oil, palm oil, medium chain triglyceride, soybean phospholipid, rapeseed oil, and a mixture thereof), animal fat (preferably, anhydrous butter, docosahexaenoic acid, and/or arachidonic acid), and 1,3-dioleoyl-2-palmitoyl triglyceride.

4. A debittered hydrolyzed protein-containing composition comprising the following components in the following parts by weight: 2-6 parts of the debittering composition according to claim 1 or 2, 2-36 parts of hydrolyzed protein (preferably, the hydrolyzed is present in an amount of 6-30 parts; and preferably, the hydrolyzed protein has a degree of hydrolysis of 5-45, preferably 6-20), 0-1 part of phospholipid, and 0-0.4 parts of an acidity regulator (preferably, the acidity regulator is one selected from the group consisting of citric acid, sodium citrate, tartaric acid, potassium citrate, malic acid, and a mixture thereof).

5. A debittered hydrolyzed protein solution comprising following components in the following parts by weight: 4-43.4 parts of the debittered hydrolyzed protein-containing composition according to any one of claims 3-5, and water as the balance, based on the total weight of the debittered hydrolyzed protein solution which is 100 parts.

6. A method for producing the debittered hydrolyzed protein solution according to claim 5, wherein the method comprises the following steps of: the components are mixed uniformly, subjected to a high speed shearing (preferably, the high speed shearing is carried out at 10000-20000 r/min and a temperature of 40-50°C for 5-20 minutes), then homogenized (preferably, the homogenization is carried out at a pressure of 30-100 MPa), and cooled to obtain the debittered hydrolyzed protein solution.

7. The producing method according to claim 6, wherein, the method further comprises a step of drying treatment to obtain a debittered hydrolyzed protein powder (preferably the drying treatment is spray drying, fluidized bed drying, rotary flash drying, microwave drying, or freeze drying), after the debittered hydrolyzed solution is obtained by cooling.

8. A debittered hydrolyzed protein powder produced by method according to claim 7.

9. A use of the composition for debittering hydrolyzed proteins according to any one of claims 1 to 3 for debittering a hydrolyzed protein.

10. A use of the composition for debittering hydrolyzed proteins according to any one of claims 1 to 3 in the manufacture of a milk powder or a formula food for a special medical use (preferably, the milk powder is an infant milk powder or a milk powder for middle-aged and old peoples; preferably, the formula food for a special medical use is an infant formula food for a special medical use).

11. An infant formula milk powder comprising a hydrolyzed protein, wherein, in the infant formula milk powder, the protein content of the infant formula is 0.45-1.2 g/100 kJ, the fat content is 0.7-1.4 g/100 kJ, and the carbohydrate content is 2.2-3.3 g/100 kJ (preferably, the infant formula milk powder at least comprises the following components in following percentages: 0.1%-40% (preferably 2%-15%) of a hydrolyzed protein (preferably, one selected from the group consisting of hydrolyzed whey protein, hydrolyzed milk protein, hydrolyzed casein, and a mixture thereof), and 10-28% of the composition for debittering hydrolyzed proteins according to any one of claims 1 to 3, optionally, the infant formula milk powder further comprises a non-hydrolyzed protein (preferably, a whey protein), and the proteins are provided by the hydrolyzed protein and milk component, or provided by the hydrolyzed protein, the milk component and the non-hydrolyzed protein).

12. A milk powder comprising a hydrolyzed protein for middle-aged and old peoples, wherein, the milk powder for middle-aged and old peoples comprises 16%-65% of proteins or 34%-69% of milk-solids-not-fat (preferably, the milk powder for middle-aged and old peoples at least comprises the following components in following percentages: 0.1%-40% (preferably 2%-15%) of a hydrolyzed protein component (preferably, one selected from the group consisting of hydrolyzed whey protein, hydrolyzed milk protein, hydrolyzed casein, and a mixture thereof), and 5-28% of the composition for debittering hydrolyzed proteins according to any one of claims 1 to 3, optionally, the milk powder for middle-aged and old peoples further comprises a non-hydrolyzed protein (preferably, a whey protein), and the proteins are provided by the hydrolyzed protein and milk component, or provided by the hydrolyzed protein, the milk component and the non-hydrolyzed protein).

13. An infant formula food for a special medical use, wherein, in the infant formula food for a special medical use, the protein content of the infant formula is 0.45-1.0 g/100 kJ, the fat content is 1.0-1.4 g/100 kJ, and the carbohydrate content is 2.2-3.3 g/100 kJ (preferably, the infant formula food for a special medical use at least comprises the following components in following percentages: 13-55% of a hydrolyzed protein component (preferably, the hydrolyzed protein component is one selected from the group consisting of hydrolyzed whey protein, hydrolyzed milk protein, hydrolyzed casein, and a mixture thereof), and 15-28% of the composition for debittering hydrolyzed proteins according to any one of claims 1 to 3.

## INTERNATIONAL SEARCH REPORT

| | International application No. |
|---|---|
| | **PCT/CN2018/109769** |

**A. CLASSIFICATION OF SUBJECT MATTER**

A23L 5/20(2016.01)i; A23C 9/00(2006.01)i; A23C 9/13(2006.01)i

According to International Patent Classification (IPC) or to both national classification and IPC

**B. FIELDS SEARCHED**

Minimum documentation searched (classification system followed by classification symbols)

A23L, A23C

Documentation searched other than minimum documentation to the extent that such documents are included in the fields searched

Electronic data base consulted during the international search (name of data base and, where practicable, search terms used)

CNABS, CPRSABS, DWPI, SIPOABS, CNTXT, CNKI, 百度学术, BAIDU ACADEMY, 读秀, DUXIU: 脱苦, 苦味, 水解蛋白, 肽, 中链, 短链, 长链, 脂肪酸, 大豆油, 玉米油, 核桃油, 棕榈油, 中链甘油三酯, 奶油, 磷脂, 二十二碳六烯酸, 花生四烯酸, 1,3-二油酸-2-棕榈酸甘油三酯, 包埋, 微胶囊, 奶粉, debitter+, bitter, hydrolyzed protein, peptide, middle chain, medium chain, short chain, long chain, fatty acid, Soybean Oil, Corn Oil, Walnut Oil, Palm oil, medium chain triglyceride, MCT, cream, Phospholipid, Ducosahexenoic Acid, DHA, Arachidonic Acid, ARA, 1,3-Oleicacid-2-Palmitaletriglyceride, OP, embed +, microcapsule

**C. DOCUMENTS CONSIDERED TO BE RELEVANT**

| Category* | Citation of document, with indication, where appropriate, of the relevant passages | Relevant to claim No. |
|---|---|---|
| X | CN 103919255 A (GUANGDONG RUNKE BIOLOGICAL ENGINEERING CO., LTD.) 16 July 2014 (2014-07-16) claims 1, 4, 5, and 9, and description, paragraph 7 | 1-9 |
| Y | CN 103919255 A (SEE ABOVE) 16 July 2014 (2014-07-16) see above | 10-13 |
| Y | CN 104703487 A (NESTEC LTD.) 10 June 2015 (2015-06-10) claims 1-14, and description, paragraphs 57 and 58 | 10-13 |
| X | CN 104883911 A (MJN U.S. HOLDINGS LLC) 02 September 2015 (2015-09-02) claims 1, 5, and 20 | 11-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 December 2018** | **15 January 2019** |

| Name and mailing address of the ISA/CN | Authorized officer |
|---|---|
| **National Intellectual Property Administration, PRC (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2018/109769**

| Patent document cited in search report | | | Publication date (day/month/year) | Patent family member(s) | | | Publication date (day/month/year) |
|---|---|---|---|---|---|---|---|
| CN | 103919255 | A | 16 July 2014 | CN | 103919225 | B | 30 September 2015 |
| CN | 104703487 | A | 10 June 2015 | AU | 2013336907 | B2 | 15 June 2017 |
| | | | | JP | 2015536136 | A | 21 December 2015 |
| | | | | CA | 2887046 | A1 | 01 May 2014 |
| | | | | US | 2015290131 | A1 | 15 October 2015 |
| | | | | EP | 2911530 | B1 | 01 February 2017 |
| | | | | WO | 2014063985 | A2 | 01 May 2014 |
| | | | | ES | 2618204 | T3 | 21 June 2017 |
| | | | | BR | 112015009002 | A2 | 04 July 2017 |
| CN | 104883911 | A | 02 September 2015 | AU | 2013372899 | B2 | 08 December 2016 |
| | | | | WO | 2014109863 | A1 | 17 July 2014 |
| | | | | US | 9301966 | B2 | 05 April 2016 |
| | | | | EP | 2943081 | A1 | 18 November 2015 |
| | | | | CA | 2897776 | A1 | 17 July 2014 |
| | | | | ES | 2649715 | T3 | 15 January 2018 |
| | | | | RU | 2015122495 | A | 17 February 2017 |
| | | | | MX | 2015008583 | A | 04 September 2015 |

Form PCT/ISA/210 (patent family annex) (January 2015)